# EUROPÄISCHE PATENTSCHRIFT

(11) **EP 3 977 530 B1**
(45) Veröffentlichungstag und Bekanntmachung des Hinweises auf die Patenterteilung: **13.08.2025**
(21) Anmeldenummer: 20726756.8
(22) Anmeldetag: 14.05.2020
(51) Int. Cl.: H10K 30/30, H10K 30/50

(54) **BISPYRANILIDENE, DITHIOBISPYRANILIDENE UND DISELENOBISPYRANILIDENE UND DEREN VERWENDUNG**
BISPYRANILIDENES, DITHIOBISPYRANILIDENES AND DISELENOBISPYRANILIDENE AND USE THEREOF
BISPYRANILIDÈNES, DITHIOBISPYRANILIDÈNES ET DISÉLÉNOBISPYRANILIDÈNES ET LEUR UTILISATION

(30) Priorität: 29.05.2019 DE 102019114456
(43) Veröffentlichungstag der Anmeldung: 06.04.2022
(73) Patentinhaber: Senorics GmbH, 01067 Dresden (DE)
(72) Erfinder: ZEIKA, Olaf, 01662 Meißen (DE); KAISER, Christina, Swansea SA1 6BS (GB); VANDEWAL, Koen, 3900 Overpelt (BE); SIEGMUND, Bernhard, 01067 Dresden (DE); BENDHUHN, Johannes, 01307 Dresden (DE); TROPIANO, Manuel, 01187 Dresden (DE)
(74) Vertreter: Kailuweit & Uhlemann Patentanwälte Partnerschaft mbB
(86) Internationale Anmeldenummer: PCT/EP2020/063434
(87) Internationale Veröffentlichungsnummer: WO 2020/239456

(56) Entgegenhaltungen:
- US-A1- 2011 083 730
- MABON ET AL.: "The cathodic coupling of heterocyclic activated thioketones. A new and efficient route to .pi.-donors. (I)-The synthesis of polysubstituted bipyranylidenes from 4H-pyran-4-thiones", NEW JOURNAL OF CHEMISTRY, vol. 13, no. 8-9, 1 January 1989 (1989-01-01), pages 601 - 607, XP009521103

## Beschreibung

Die Erfindung betrifft Bispyranilidene, Dithiobispyranilidene und Diselenobispyranilidene gemäß Formel (I), deren Verwendung als Licht- oder IR-Absorber und ein elektronisches oder optoelektronisches Bauelement enthaltend mindestens eine Verbindung gemäß Formel (I).

Optoelektronische Bauelemente haben die Eigenschaft entweder Licht, insbesondere Sonnenlicht, in Elektrizität umzuwandeln oder umgekehrt. Diese Bauelementklasse umfasst Solarzellen, OLEDs und Sensoren, insbesondere Photodetektoren. Solarzellen sind optimiert, um einen größtmöglichen Anteil des Sonnenlichtes in elektrische Leistung umzuwandeln. Detektoren hingegen werden häufig mit einer extern angelegten Spannung betrieben, um höhere externe Quanteneffizienzen im Detektionsbereich und schnellere Reaktionszeiten zu erzielen. Der Detektionsbereich kann im sichtbaren und außerhalb des sichtbaren Lichtes liegen.

Elektronische Bauelemente, wie organische Feldeffekttransistoren (OFETs) können neben den drei Kontakten (Source, Drain, Gate) aus einem sehr dünnen Isolatorfilm und einer organischen Halbleiterschicht bestehen, deren Drain-Spannungs/Stromkennlinien wesentlich von der Gate-Spannung abhängen.

Die Entwicklung von Verbindungen zur Verwendung in elektronischen und optoelektronischen Bauelementen ist aktuell Gegenstand intensiver Forschung. Ziel ist die Entwicklung und Untersuchung von Verbindungen, welche ein verbessertes Eigenschaftsprofil, insbesondere hinsichtlich der Absorptionsbereiche, der elektrischen Mobilität und damit der Bauteileffizienzen, ermöglichen.

Strzelecka et al. offenbaren die Untersuchung der optischen und elektrischen Eigenschaften von unsubstituierten und mittels Methylgruppe-, Arylgruppe-, oder Thiophengruppe substituierten Dipyranylidenen und Dithiopyranylidenen (Strzelecka et al. 1979). Die Verbindungen werden zur Herstellung von TCNQ-Molekülkomplexen verwendet.

Fabre et al. offenbaren Dipyranylidene, substituiert mit verschiedenen aromatischen Resten, insbesondere mit Phenyl-, p-Tolyl- oder Thiophengruppen (Fabre et al. 1976).

Mabon et al. beschreiben ein Verfahren zur Herstellung von verschiedenen tetrasubstituierten Dipyranylidenen, insbesondere symmetrischen und unsymmetrischen Dipyranylidenen, substituiert mit Methyl- oder Arylgruppen, wie Phenyl-, Thiophen- oder Anisylgruppen (Mabon et al. 1989).

Eine Variante zur Verbesserung der Eigenschaftsprofile optoelektronischer Bauteile, wie Photovoltaikzellen und Photodetektoren, insbesondere Nahinfrarot (NIR)- und Infrarot (IR)-Sensoren, bietet die Nutzung von sogenannten Charge-Transfer-Übergängen. Charge-Transfer-Übergänge (CT, Ladungstransfer-Übergänge) werden in intermolekulare und intramolekulare Charge-Transfer-Übergänge unterschieden. Bei einem Ladungstransfer-Übergang handelt es sich um eine vollständige oder nahezu vollständige Ladungsübertragung von einer Donorverbindung auf eine Akzeptorverbindung. Sind beide Verbindungen im gleichen Molekül verankert, so liegt ein intramolekularer Ladungstransfer-Übergang vor. Wirken verschiedene (diskrete) Moleküle oder Ionen, die auch lose durch koordinative Wechselwirkung gekoppelt sein können, als Donorverbindung und Akzeptorverbindung, so spricht man von einem intermolekularen Ladungstransfer. Der intermolekulare Ladungstransfer-Übergang führt typischerweise zu elektrostatisch gebundenen Donor-Akzeptor-Komplexen.

Ein intermolekularer Ladungstransferzustand ist ein schwach gebundener Zustand zwischen einem angeregten Elektron im LUMO (oder einem energetisch höher liegenden Zustand) sowie einem Loch im HOMO (oder energetisch niedriger), wobei das genannte Loch und Elektron sich auf räumlich getrennten Molekülen befinden. Vorzugsweise bildet sich der Ladungstransferzustand an der Grenzfläche zwischen Donorverbindung und Akzeptorverbindung aus. Anschließend überträgt die, in Folge der Absorption elektromagnetischer Strahlung angeregte, Donorverbindung die negative Ladung vom LUMO über einen interchromophoren Ladungstransfer auf das LUMO der Akzeptorverbindung oder geht durch Rekombination in den Grundzustand über.

EP 3 152 785 B1 und Siegmund *et al.* beschreiben Nahinfrarot-Photodetektoren basierend auf intermolekularer Charge-Transfer-Absorption (Siegmund *et al.* 2017), insbesondere die Nutzung optischer Mikrokavitäten zur mehr als 40-fachen Erhöhung der typischerweise vernachlässigbaren äußeren Quanteneffizienz (EQE) im Spektralbereich der Charge-Transfer-Absorption. Siegmund *et al.* beschreiben EQEs über 20% sowie Spektrallinienbreiten bis hinab auf 36 nm und Resonanzwellenlängen zwischen 810 nm und 1550 nm basierend auf Mischungen von C₆₀-Fulleren und Donormaterialien mit hohen HOMO-Niveaus, insbesondere ZnPc:C₆₀ und TPDP:C₆₀.

EP 3 152 785 B1 beschreibt die Detektion eines elektromagnetischen Signals im spektralen Wellenlängenbereich von 780 nm bis 10 µm unter Verwendung von Donorverbindungen bevorzugt ausgewählt aus der Stoffgruppe der Phthalocyanine, wie Zinkphthalocyanin oder Eisenphthalocyanin; der Pyrane, wie Bispyranilidene, insbesondere TPDP; der Fulvalene, wie Tetrathiofulvalen, oder der aromatischen Amine, wie N,N,N',N'-Tetrakis(4-methoxyphenyl)-benzidine, 2,7-Bis[N,N-bis(4-methoxy-phenyl)amino]9,9-spiro-bifluorene oder 4,4',4"-Tris(3-methylphenyl-phenylamino)triphenylamin), der Bisthiopyranilidene, der Bipyridinylidene oder der Diketopyrrolopyrrole und einer Akzeptorverbindung bevorzugt ausgewählt aus Fullerenen, wie C₆₀.

Bolag *et al.* offenbaren Feldeffekttransistoren basierend auf Tetraphenyldipyranilidenen (TPDP) und deren Schwefelanaloga (Bolag *et al.* 2009). Bolag *et al.* beschreiben die Stabilität der Kationen und Dikationen von Tetraphenyldipyranilidenen, das ausgedehnte π-System, welches vorteilhaft für intermolekulare Wechselwirkungen ist, ein einfaches Herstellungsverfahren und eine hohe Absorption im sichtbaren Bereich. Weiterhin offenbaren Bolag *et al.* eine höhere Leistung der Schwefelverbindung gegenüber Tetraphenyldipyraniliden aufgrund der höheren Kristallinität und die Einführung von Halogensubstituenten zur Erhöhung der Stabilität und Löslichkeit.

DE 10 2015 101 768 A1 offenbart unsubstituierte und substituierte chinoide Aromaten, Polyaromaten, Heteroaromaten oder Polyheteroaromaten, deren mögliche Verwendung in einem optoelektronischen Bauelement sowie das optoelektronische Bauelement und dessen Verwendung als IR-Absorber in Folien und dünnen Schichten, insbesondere in Wärmeschutzverglasungen.

US 2011 / 0 083 730 A1 offenbart symmetrische und unsymmetrische Verbindungen der Formel (I) 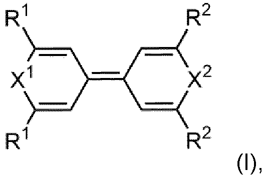 wobei X¹ und X² unabhängig voneinander aus N, P, O, S, Se und Te ausgewählt sind und R¹ und R² unabhängig voneinander aus unsubstituierten oder substituierten Aromaten und Heteroaromaten mit 4 bis 10 C-Atomen ausgewählt sind, sowie deren Verwendung in elektronischen und optoelektronischen Bauteilen. Als Heteroaromat werden Thiophenyl- und Alkoxythiophenylreste, insbesondere 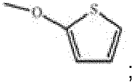 Furyl-, Pyrrolyl-, Pyridyl-, Pyrazyl-, Pyrazolyl-, Pyridazyl-, Pyrimidyl-, Triazyl-, Imidazolyl-, Oxazolyl-, Indyl-, Indazolyl-, Quinolyl- und Quinoxalylreste offenbart.

Trotz der durch verschiedene Ansätze erreichten verbesserten Effizienzen für optoelektronische Bauelemente sind die derzeitig erreichten Effizienzen und Bauelement-Lebensdauern noch nicht ausreichend für einen kommerziellen Einsatz.

Daher besteht die Aufgabe der vorliegenden Erfindung darin, organische, photoaktive Verbindungen bereitzustellen, welche eine hohe Absorptionsintensität aufweisen.

Ferner ist es Aufgabe der Erfindung, elektronische oder optoelektronische Bauelemente mit hohen Effizienzen bereitzustellen.

Erfindungsgemäß wird die Aufgabe gelöst durch eine Verbindung gemäß Formel (I) 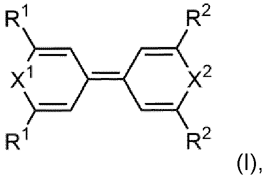
wobei X¹ und X² jeweils unabhängig voneinander aus der Gruppe umfassend Sauerstoff, Schwefel und Selen ausgewählt sind, wobei R¹ und R² jeweils unabhängig voneinander aus der Gruppe umfassend 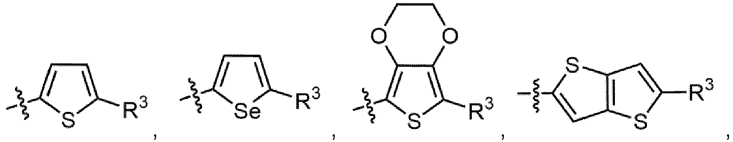 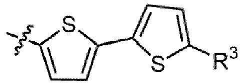
ausgewählt sind, wobei R³ ausgewählt ist aus C1- bis C20-Alkylresten.

Vorteilhaft weisen die erfindungsgemäßen Verbindungen eine hohe thermische Stabilität auf. Vorteilhaft sind die erfindungsgemäßen Verbindungen im Hochvakuum sublimierbar. Weiterhin vorteilhaft weisen die erfindungsgemäßen Verbindungen eine Photoaktivität im sichtbaren und Infrarotbereich auf. Vorteilhaft sind die erfindungsgemäßen Verbindungen Elektronendonorverbindungen und besonders vorteilhaft weisen die erfindungsgemäßen Verbindungen mit geeigneten p-Akzeptormaterialien langwellige und intensive absorbierende Charge-Transfer (CT, Ladungstransfer)-Übergänge auf. Der Grund für die Verschiebung der Absorption der erfindungsgemäßen Verbindungen mit substituierten Thiophen- und Selenophenresten über den CT-Zustand in den weiter roten bzw. langwelligeren Absorptionsbereich liegt in der Weitung und Erhöhung des HOMO-Zustandes, wodurch die Überlappung mit den LUMO-Energien größer wird und damit auch die Überlappung mit dem verwendeten Akzeptor.

Erfindungsgemäß sind R¹ und R² jeweils unabhängig voneinander aus der Gruppe umfassend 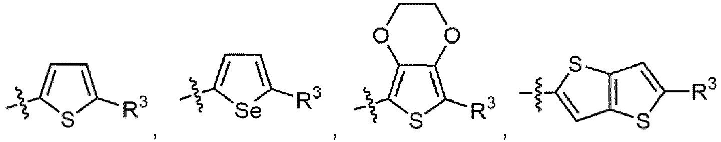 und 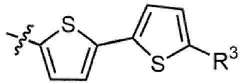 ausgewählt, wobei R³ aus C1- bis C20-Alkylresten ausgewählt ist.

In weiteren Ausführungsformen ist R³ aus der Gruppe umfassend Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl ausgewählt.

Erfindungsgemäß ist R³ ausgewählt aus der Gruppe umfassend unsubstituierte und substituierte C1- bis C20-Alkylreste, besonders bevorzugt aus der Gruppe umfassend Methyl, Ethyl, Propyl, Butyl, Pentyl und Hexyl.

In einer Ausführungsform sind R¹ und R² identisch.

In weiteren Ausführungsformen sind X¹ und X² Sauerstoff und Schwefel, Sauerstoff und Selen oder Schwefel und Selen.

In bevorzugten Ausführungsformen sind X¹ und X² jeweils unabhängig voneinander aus der Gruppe umfassend Schwefel und Selen ausgewählt.

In einer bevorzugten Ausführungsform sind X¹ und X² identisch. In einer bevorzugten Ausführungsform sind X¹ und X² aus der Gruppe umfassend Sauerstoff, Schwefel und Selen ausgewählt, besonders bevorzugt sind X¹ und X² Schwefel.

In weiteren Ausführungsformen sind die erfindungsgemäßen Verbindungen symmetrisch, wobei R¹ und R² und X¹ und X² identisch sind.

Besonders bevorzugte Ausführungsformen der erfindungsgemäßen Verbindungen sind die folgenden Einzelverbindungen:
2,2',6,6'-Tetra-(2-methylthienyl)-4,4'-bispyraniliden,
2,2',6,6'-Tetra-(2-ethylthienyl)-4,4'-bispyraniliden,
2,2',6,6'-Tetra-(2-propylthienyl)-4,4'-bispyraniliden,
2,2',6,6'-Tetra-(2-butylthienyl)-4,4'-bispyraniliden,
2,2',6,6'-Tetra-(2-pentylthienyl)-4,4'-bispyraniliden,
2,2',6,6'-Tetra-(2-hexylthienyl)-4,4'-bispyraniliden,
2,2',6,6'-Tetrakis(7-methyl-2,3-dihydrothieno[3,4-b][1,4]dioxin-5-yl)-4,4'-bispyraniliden,
2,2',6,6'-Tetra-(2-heptylthienyl)-4,4'-bispyraniliden,
2,2',6,6'-Tetra-(2-methylthienyl)-4,4'-dithiobispyraniliden,
2,2',6,6'-Tetra-(2-ethylthienyl)-4,4'-dithiobispyraniliden,
2,2',6,6'-Tetra-(2-propylthienyl)-4,4'-dithiobispyraniliden,
2,2',6,6'-Tetra-(2-butylthienyl)-4,4'-dithiobispyraniliden,
2,2',6,6'-Tetra-(2-pentylthienyl)-4,4'-dithiobispyraniliden,
2,2',6,6'-Tetra-(2-hexylthienyl)-4,4'-dithiobispyraniliden,
2,2',6,6'-Tetra-(2-heptylthienyl)-4,4'-dithiobispyraniliden,
2,2',6,6'-Tetrakis(7-methyl-2,3-dihydrothieno[3,4-b][1,4]dioxin-5-yl)-4,4'-dithiobispyraniliden,
2,2',6,6'-Tetra-(2-methylthienyl)-4,4'-diselenobispyraniliden,
2,2',6,6'-Tetra-(2-ethylthienyl)-4,4'-diselenobispyraniliden,
2,2',6,6'-Tetra-(2-propylthienyl)-4,4'-diselenobispyraniliden,
2,2',6,6'-Tetra-(2-butylthienyl)-4,4'-diselenobispyraniliden,
2,2',6,6'-Tetra-(2-pentylthienyl)-4,4'-diselenobispyraniliden,
2,2',6,6'-Tetra-(2-hexylthienyl)-4,4'-diselenobispyraniliden,
2,2',6,6'-Tetra-(2-heptylthienyl)-4,4'-diselenobispyraniliden oder
2,2',6,6'-Tetrakis(7-methyl-2,3-dihydrothieno[3,4-b][1,4]dioxin-5-yl)-4,4'-diselenobispyraniliden.

Gegenstand der Erfindung ist auch die Verwendung mindestens einer Verbindung gemäß Formel (I), wobei X¹ und X² jeweils unabhängig voneinander aus der Gruppe umfassend Sauerstoff, Schwefel und Selen ausgewählt sind, wobei R¹ und R² jeweils unabhängig voneinander aus der Gruppe umfassend 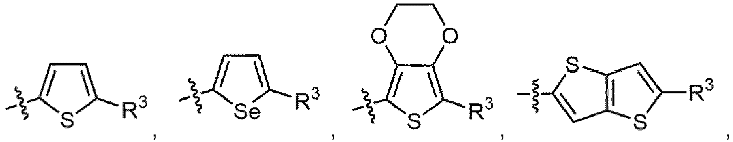 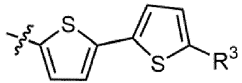 ausgewählt sind, wobei R³ ausgewählt ist aus C1- bis C20-Alkylresten, als Licht- oder IR-Absorber, insbesondere in einem elektronischen oder optoelektronischen Bauelement.

Unter "Licht" wird eine elektromagnetische Strahlung mit einer Wellenlänge im Bereich von 380 nm bis 780 nm verstanden. Unter Infrarot (IR) wird eine elektromagnetische Strahlung mit einer Wellenlänge im Bereich von 780 nm bis 1 mm verstanden. Unter "Nahem Infrarot (NIR)" wird eine elektromagnetische Strahlung im Wellenlängenbereich von 780 nm bis 3 µm verstanden.

Unter einem "Licht- oder IR-Absorber" wird eine Verbindung verstanden, welche zumindest einen Teil der elektromagnetischen Strahlung mit Wellenlängen im Bereich von 380 nm bis 1 mm absorbieren kann.

Unter einem "optoelektronischen Bauelement" wird ein Bauelement verstanden, welches eine Schnittstelle zwischen elektrischen und optischen Komponenten darstellt In Ausführungsformen sind optoelektronische Bauelement aus der Gruppe umfassend organische Solarzellen *(organic solar cells*,OSC), Grätzelzellen, organische integrierte Schaltungen (*organic integrated circuits,* O-IC), organische Feld-Effekt-Transistoren (OFETs), organische Dünnfilmtransistoren *(organic thin film transistor,* O-TFT), organische Leuchtdioden (*organic light emitting diode,* OLEDs), Photodetektoren und IR-Sensoren, insbesondere Infrarot (IR)-*Charge-Transfer* (CT)-Absorptionssensoren, ausgewählt.

In verschiedenen Ausführungsformen erfolgt die Verwendung mindestens einer erfindungsgemäßen Verbindung als Licht- oder IR-Absorber in Kombination mit Elektronenakzeptoren, bevorzugt Fullerenen, besonders bevorzugt C₆₀- oder C₇₀-Fullerenen; oder Fullerenderivaten, wie 1-(3-Methoxycarbonyl)-Propyl-1-1-Phenyl-(6,6)C₆₁ (PCBM) als photoaktive Mischschichten in optoelektronischen Bauelementen. Vorteilhaft erfolgt nach der Absorption von Licht oder IR-Strahlung durch eine erfindungsgemäße Verbindung eine Übertragung von Elektronen auf den Elektronenakzeptor. Bevorzugt gelangen die Elektronen über eine Elektronentransportschicht zur Elektrode.

In weiteren Ausführungsformen erfolgt die Verwendung als Donorabsorbermaterial in organischen Solarzellen (*organic solar cells,OSC*), als Lochtransportmaterial (*hole transport material,* HTM) in Grätzelzellen, in organischen integrierten Schaltungen (*organic integrated circuits,* O-IC), in organischen Feld-Effekt-Transistoren (OFETs), in organischen Dünnfilmtransistoren *(organic thin film transistor,* O-TFT), in organischen Leuchtdioden *(organic light emitting diode,* OLEDs), Photodetektoren oder in IR-Sensoren, insbesondere Infrarot (IR)-*Charge-Transfer* (CT)-Absorptionssensoren.

Bevorzugt erfolgt die Verwendung mindestens einer erfindungsgemäßen Verbindung in Infrarot (IR)-*Charge-Transfer* (CT)-Absorptionssensoren. Vorteilhaft erreichen IR-CT-Absorptionssensoren mit den erfindungsgemäßen Verbindungen Spektrallinienbreiten von maximal 100 nm, bevorzugt maximal 50 nm, besonders bevorzugt maximal 15 nm.

Gegenstand der Erfindung ist auch die Verwendung mindestens einer Verbindung gemäß Formel (I), wobei X¹ und X² jeweils unabhängig voneinander aus der Gruppe umfassend Sauerstoff, Schwefel und Selen ausgewählt sind, wobei R¹ und R² jeweils unabhängig voneinander aus der Gruppe umfassend 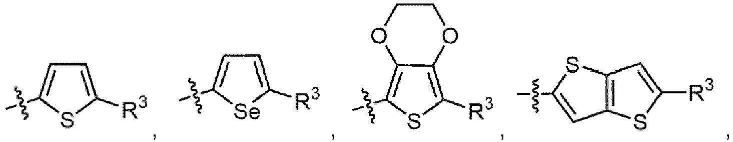 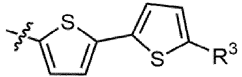 ausgewählt sind, wobei R³ ausgewählt ist aus C1- bis C20-Alkylresten, in einem Verfahren zur Detektion eines elektromagnetischen Signals im Wellenlängenbereich von 780 nm bis 10 µm.

Bevorzugt erfolgt die Verwendung in einem Verfahren zur Detektion eines elektromagnetischen Signals im Wellenlängenbereich von 780 nm bis 10 µm aufweisend die Schritte:
a) Bereitstellung eines optoelektronischen Bauelements, das auf einem Substrat angeordnet ist und
   i. zwei voneinander getrennte und sich gegenüberliegende Spiegelflächen aufweist, die eine optische Mikrokavität ausbilden,
   ii. eine zwischen den Spiegelflächen angeordnete photoaktive Schicht aufweist, enthaltend mindestens eine Verbindung gemäß Formel (I) und eine weitere Verbindung,
      wobei die weitere Verbindung vorzugweise aus Fullerenen, bevorzugt C₆₀- oder C₇₀-Fullerenen, oder Fullerenderivaten, bevorzugt 1-(3-Methoxycarbonyl)-Propyl-1-1-Phenyl-(6,6)C₆₁ (PCBM), ausgewählt ist, wobei die Energiedifferenz zwischen der HOMO-Energie der Verbindung gemäß Formel (I) und der LUMO-Energie der weiteren Verbindung unterhalb 1,6 eV liegt,

   wobei die optische Weglänge zwischen den Spiegelflächen im Bereich von 25 bis 75% der Wellenlänge des zu detektierenden Signals entspricht, und
   wobei das Energieäquivalent des Wellenlängenbereichs des zu detektierenden elektromagnetischen Signals im Bereich von
      - der Energiedifferenz definiert durch die HOMO-Energie der Verbindung gemäß Formel (I) und die LUMO-Energie der weiteren Verbindung und
      - der Energiedifferenz definiert durch die HOMO-Energie und die LUMO-Energie der Verbindung gemäß Formel (I) liegt,
   wobei die photoaktive Schicht innerhalb der optischen Mikrokavität im räumlichen Intensitätsmaximum der Wellenlänge des zu detektierenden elektromagnetischen Signals zwischen den Spiegelflächen ausgerichtet ist;
b) Bestrahlen des optoelektronischen Bauelements mit einem elektromagnetischen Signal im Wellenlängenbereich von 780 nm bis 10 mm;
c) Verstärkung des zu detektierenden elektromagnetischen Signals innerhalb der optischen Mikrokavität, wobei durch die Wellenlänge des zu detektierenden Signals induziert, ein direkter interchromophorer Ladungstransfer von der Verbindung gemäß Formel (I) auf die weitere Verbindung erfolgt;
d) Umwandlung des elektromagnetischen Signals in ein elektrisches Signal.

Ein weiterer Aspekt der Erfindung betrifft ein elektronisches oder optoelektronisches Bauelement enthaltend mindestens eine Verbindung gemäß Formel (I), wobei X¹ und X² jeweils unabhängig voneinander aus der Gruppe umfassend Sauerstoff, Schwefel und Selen ausgewählt sind, wobei R¹ und R² jeweils unabhängig voneinander aus der Gruppe umfassend 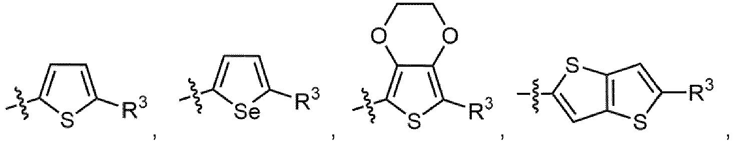 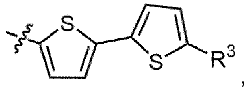 ausgewählt sind, wobei R³ ausgewählt ist aus C1- bis C20-Alkylresten.

In Ausführungsformen umfassen die optoelektronischen Bauelemente weiterhin mindestens eine weitere Verbindung, insbesondere eine Elektronenakzeptorverbindung (Akzeptoverbindung), wobei die weitere Verbindung vorzugweise aus Fullerenen, bevorzugt C₆₀- oder C₇₀-Fullerenen, oder Fullerenderivaten, bevorzugt 1-(3-Methoxycarbonyl)-Propyl-1-1-Phenyl-(6,6)C₆₁ (PCBM), ausgewählt ist. Bevorzugt weist das optoelektronische Bauelement mindestens eine erfindungsgemäße Verbindung und mindestens eine weitere Verbindung als photoaktive Mischschicht auf.

In weiteren Ausführungsformen weist das optoelektronische Bauelement, insbesondere eine organische Solarzelle, zwei oder mehrere photoaktive Schichten Multijunction-Bauteile) auf, wobei die photoaktiven Schichten zumeist in einzelnen meist vertikal direkt übereinander prozessierten Solarzellen untergebracht sind, die über einen sogenannten Rekombinationskontakt in Serie verschaltet werden.

In verschiedenen Ausführungsformen weist das optoelektronische Bauelement, insbesondere eine organische Solarzelle, die erfindungsgemäßen Verbindungen als Lichtabsorber in sogenannten Kaskadenstrukturen auf. Dabei besteht die photoaktive Schicht der Solarzelle aus einer Abfolge von mehreren Donatormolekülen gefolgt von mehreren Akzeptormolekülen (je nach Ausführung als p-i-n- oder n-i-p-Struktur auch in umgekehrter Reihenfolge). In anderen Ausführungsformen können auch mehrere Donatoren mit mehreren Akzeptoren gemischt werden, um die photoaktive Schicht zu bilden und damit einen breiteren Spektralbereich des Sonnenlichtes abzudecken.

Vorteilhaft ist die Intensität des Charge-Transfers des optoelektronischen Bauelements zwei- bis dreimal größer als von optoelektronischen Bauelementen umfassend Tetraphenyldipyraniliden (TPDP).

In einer Ausführungsform weisen die erfindungsgemäßen optoelektronischen Bauelemente einen Absorptionsbereich mit Wellenlängen bis mindestens 1000 nm, bevorzugt bis mindestens 1300 nm, besonders bevorzugt bis mindestens 1600 nm, auf.

In bevorzugten Ausführungsformen weisen die erfindungsgemäßen optoelektronischen Bauelemente einen Absorptionsbereich von 810 nm bis 1665nm, bevorzugt von 900 nm bis 1300 nm, auf.

In weiteren Ausführungsformen umfasst das optoelektronische Bauelement Elektroden bestehend aus Metall, einem leitfähigen Oxid, insbesondere Indiumzinnoxid *(indium tin oxide,* ITO), ZnO:Al oder einem anderen transparenten, elektrisch leitfähigen Oxid *(transparent conductive oxide,* TCO); oder einem leitfähigen Polymer, insbesondere PEDOT/PSS (Poly(3,4-ethylenedioxy-thiophene)po-(styrenesulfonate)) oder PANI (Polyanilin).

In verschiedenen Ausführungsformen ist die Elektrode, welche auf einem Substrat angeordnet ist, für Licht transluzent ausgeführt. Unter "Transluzenz" wird eine partielle Lichtdurchlässigkeit eines Materials verstanden, zumindest in einem gewissen Lichtwellenlängenbereich mit einer Durchlässigkeit im Bereich von 1% bis 100%.

In Ausführungsformen weist das optoelektronische Bauelement zumindest:
i. zwei voneinander getrennte und sich gegenüberliegende Spiegelflächen, die eine optische Mikrokavität ausbilden,
ii. eine zwischen den Spiegelflächen angeordnete photoaktive Schicht, enthaltend mindestens eine Verbindung gemäß Formel (I) und eine weitere Verbindung, wobei die weitere Verbindung vorzugweise aus Fullerenen, bevorzugt C₆₀- oder C₇₀-Fullerenen, oder Fullerenderivaten, bevorzugt 1-(3-Methoxycarbonyl)-Propyl-1-1-Phenyl-(6,6)C₆₁ (PCBM), ausgewählt ist, wobei die Energiedifferenz zwischen der HOMO-Energie der Verbindung gemäß Formel (I) und der LUMO-Energie der weiteren Verbindung unterhalb 1,6 eV liegt,

wobei die optische Weglänge zwischen den Spiegelflächen im Bereich von 25 bis 75% der Wellenlänge des zu detektierenden Signals entspricht und
wobei das Energieäquivalent des Wellenlängenbereichs des zu detektierenden elektromagnetischen Signals im Bereich von
   - der Energiedifferenz definiert durch die HOMO-Energie der Verbindung gemäß Formel (I) und die LUMO-Energie der weiteren Verbindung und
   - der Energiedifferenz definiert durch die HOMO-Energie und die LUMO-Energie der Verbindung gemäß Formel (I) liegt,
wobei die photoaktive Schicht innerhalb der optischen Mikrokavität im räumlichen Intensitätsmaximum der Wellenlänge des zu detektierenden elektromagnetischen Signals zwischen den Spiegelflächen ausgerichtet ist,
auf einem Substrat auf.

Ein weiterer Aspekt der Erfindung betrifft die Verwendung des optoelektronischen Bauelements für die Detektion eines elektromagnetischen Signals im Wellenlängenbereich von 780 nm bis 10 µm mit räumlicher, zeitlicher und/oder spektraler Auflösung sowie zu deren Weiterverarbeitung.

Für die Realisierung der Erfindung ist es auch zweckmäßig, die vorbeschriebenen Ausführungsformen und Merkmale der Ansprüche zu kombinieren, insbesondere die vorbeschriebenen Ausführungsformen der erfindungsgemäßen Verbindung auf die beschriebene Verwendung und das beschriebene elektronische oder optoelektronische Bauelement anzuwenden.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

Es zeigen die
**Fig. 1** die UV-Vis-Absorptionsspektren von Thienyl-substituierten Bispyranylidenen und Dithiobispyranilidenen in Dimethylformamid (DMF) (c = 10⁻⁵ mol/I).
**Fig. 2** die Messung der externen Quanteneffizienz (EQE) und internen Quanteneffizienz (IQE) der optoelektronischen Bauteile umfassend 2,2',6,6'-Tetrathienyl-4,4'-dithiobispyranyliden (Referenz) (Kreise) oder 2,2',6,6'-Tetra(2-methylthienyl)-4,4'-dithiobispyranyliden (Quadrate) und C₆₀. Unausgefüllte Zeichen stellen die IQE im Spektralbereich von 425 nm bis 525 nm dar (IQE = EQE • Absorption ⁻¹). Die gestrichelte Linie stellt die mittlere IQE dar. Ausgefüllte Zeichen stellen die EQE dar.
**Fig. 3** **a)** die CT-Absorptionsprofile *σ*_{CT} der Mischungen der Verbindungen mit C₆₀ (lineare und logarithmische Skalierung). **b)** Oszillatorstärke *f*_{σ}, **c)** Struktur der symmetrischen Thienyl-substituierten Bispyranylidenen und Dithiobispy-ranilidenen.

### Allgemeine synthetische Arbeitstechniken

Die Lösungsmittel werden vor der Verwendung entsprechend Standardtechniken gereinigt und getrocknet.

### Elektronenspray-Ionisation-Massenspektrometrie (Electron Spray Ionization-Mass Spectrometry, ESI-MS)

Massenspektrometrie wird mittels Bruker Esquire Ion Trap (ESI/APCI) und einer Probenkonzentration von 2 mg/l durchgeführt.

### Kernspinresonanz (Nuclear Magnetic Resonance, NMR)-Spektroskopie

Die Messung der NMR-Spektren wird entweder mit einem Bruker AC 300, AC-600 oder einem Bruker DRX 500 Kernresonanzspektrometer in deuterierten Lösungsmitteln bei 26 bis 30°C durchgeführt. Verschiebungen der¹H- und ¹³C-Resonanzen sind in ppm relativ zu dem Restsignal des nicht-deuterierten Lösungsmitteln angegeben. Kopplungskonstanten werden ohne Angabe des Vorzeichens in Hz angegeben, wobei für die Multiplizitäten der einzelnen Signale folgende Abkürzungen benutzt werden: s: Singulett; d: Dublett; dd: Duplett vom Duplett; t: Triplett; qua: Quartett; qi: Quintett; sep: Septett; m: Multiplett; br.s.: breites Signal.

### Ultraviolet-Visible (UV-Vis) Absorption-Spektroskopie

Die optische Charakterisierung erfolgt mittels UV-Vis-Spektroskopie zur Bestimmung der optischen Bandbreite, die Form der Absorptionsbande und des Extinktionskoeffizienten. UV-Vis-Spektren werden mittels Perkin Elmer Lambda 25 UV/VIS-Spektrophotometer mit einer Scanrate von 600 nm/min gemessen.

### Synthesevorschrift für 1,5-Di-(2-thienyl)pentan-1,5-dion

15 ml wasserfreies Dichlormethan (DCM) wird unter Schutzgasatmosphäre zu 16,0 g (120 mmol, 2 Äq.) Aluminiumchlorid in einem 100 ml-Rundkolben gegeben. Tropfenweise wird eine Lösung aus 11,6 ml (120 mmol, 2,4 Äq.) Thiophen und 6,4 ml (50 mmol, 1 Äq.) Glutarylchlorid in 15 ml DCM über 10 min zugegeben. Bei der Zugabe ändert sich die Farbe von hellorange zu dunkelrot. Die Lösung wird über Nacht gerührt und der Kolben in einem Eisbad abgekühlt. Die Reaktion wird mittels Eis und konzentrierter Salzsäure (2 ml) gestoppt. Unter Rühren wird Wasser zugegeben bis die exotherme Reaktion mit dem Überschuss Aluminiumchlorid beendet ist. Die Mischung wird mit 50 ml DCM verdünnt und für 2 h gerührt. Die organische Phase wird mit warmen DCM extrahiert, über Magnesiumsulfat getrocknet und unter Vakuum konzentriert. Das Rohprodukt wird zermahlen und mit kaltem Diethylether gewaschen. 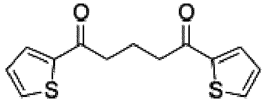
Summenformel: C₁₃H₁₂O₂S₂ (264,03 g/mol)
Ausbeute: 11,2 g (42,3 mmol, 85 %)
ESI-MS: m/z 265 [M]⁺
¹H-NMR (500 MHz, CDCl₃, ppm): δ = 7.73 (dd, J = 3.8, 1.1 Hz, 1H), 7.61 (dd, J = 4.9, 1.1 Hz, 1H), 7.10 (dd, J = 4.9, 3.8 Hz, 1H), 3.04 (t, J = 7.0 Hz, 2H), 2.19 (qt, J = 7.0, 3.5 Hz, 1H).
¹³C-NMR (75 MHz, CDCl₃, ppm): δ = 193.39, 144.81, 134.22, 132.68, 128.78, 38.81, 19.96.

### Synthesevorschrift für 2,6-Di-(2-thienyl)pyryliumtetrafluoroborat

9,7 ml (76,2 mmol, 10 Äq.) Tetrafluorborsäurelösung (50 % (m/m) in Wasser) werden tropfenweise über 30 Minuten zu einer Suspension aus 2,0 g (7,6 mmol, 1 Äq.) 1,5-Di-(2-thienyl)pentan-1,5-dion in 50 ml Essigsäureanhydrid gegeben, während die Temperatur mittels eines Eisbads unterhalb 15°C gehalten wird. Nachdem die Zugabe abgeschlossen ist, wird die Mischung für weitere 2 h bei Raumtemperatur gerührt und über Nacht bei 5°C stehen gelassen. Nach der Zugabe von 500 ml Hexan/Diethylether (1:10) fällt ein brauner Niederschlag aus. Das Produkt wird durch Vakuumfiltration, Waschen mit Diethylether und Vakuumtrocknung bei Raumtemperatur erhalten. 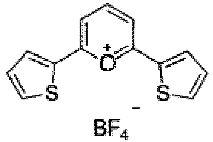
Summenformel: C₁₃H₉BF₄OS₂ (332,01 g/mol)
Ausbeute: 1,59 g (4,8 mmol, 63 %)
ESI-MS: m/z 245 [M-BF₄]⁺, 277 [M-BF₄+CH₃OH]⁺
Absorption (DCM): αₘₐₓ = 489 nm (ε = 30458 Lmol⁻¹cm⁻¹)
¹H-NMR (500 MHz, Acetonitril, ppm): δ = 8.61 (t, J = 8.4 Hz, 1H), 8.28 (dd, J = 4.0, 1.1 Hz, 2H), 8.21 (dd, J = 4.9, 1.1 Hz, 2H), 8.08 (d, J = 8.4 Hz, 2H), 7.44 (dd, J = 4.9, 4.1 Hz, 2H).
¹³C-NMR (75 MHz, Acetonitril, ppm): δ = 167.00, 155.90, 140.54, 186.98, 188.28, 181.95, 117.71.

### Synthesevorschrift für 2,2',6,6'-Tetrathienyl-4,4'-bispyranyliden

Unter Schutzgasatmosphäre werden 1,2 ml (4,7 mmol, 1 Äq.) Tributylphosphin zu einer orangen Suspension aus 1,56 g (4,7 mmol, 1 Äq.) 2,6-Di-(2-thienyl)pyryliumtetrafluoroborat in 50 ml getrocknetem Acetonitril gegeben. Die Mischung ändert die Farbe zu gelb und wird für 2,5 h bei Raumtemperatur gerührt. Anschließend werden 4,0 ml (23,5 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird als schwarzer Feststoff nach Filtrieren, Waschen mit Acetonitril und Trocknen an Luft erhalten. 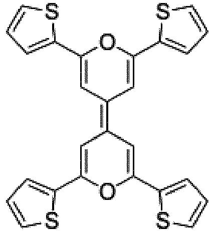
Summenformel: C₂₆H₁₆O₂S₄ (488,00 g/mol)
Ausbeute: 0,62 g (1,27 mmol, 54 %)
ESI-MS: m/z 488 [M]⁺
Absorption (DMF): αₘₐₓ = 482 nm (ε = 29610 Lmol⁻¹cm⁻¹)
Schmelzpunkt: 239 °C
¹H-NMR (500 MHz, CDCl₃, ppm): δ = 7.74 (dd, J = 3.7, 1.1 Hz, 1H), 7.65 (dd, J = 5.0, 1.1 Hz, 1H), 7.21 (dd, J = 5.0, 3.7 Hz, 1H), 6.95 (s, 1H).
¹³C-NMR (125.75 MHz, CDCl₃, ppm): δ = 144.90, 136.50, 12.08, 126.42, 124.15, 113.68, 101.85.

### Synthesevorschrift für 2,6-Dithienylthiopyryliumperchlorat

In einen 250 ml-Rundkolben werden sukzessiv 7,80 g (29,5 mmol, 1,0 Äq.) 1,5-Di-(2-thienyl)pentan-1,5-dion, 9,86 g (44,3 mmol, 1,5 Äq.) Phosphor(V)-sulfid, 180 ml Essigsäure und 18,90 g (60 mmol, 6,0 Äq.) Lithiumperchlorat eingebracht. Die Mischung wird für 3 h unter Rückfluss gekocht. Die Farbe wechselt von orange zu dunkelrot. Ein grüner Feststoff wird durch Filtration und Waschen mit heißer Essigsäure erhalten. Das Filtrat wird unter Vakuum konzentriert und durch Zusatz eines Überschusses Diethylether und Lagerung bei 5°C über Nacht wird ein schwarzer Feststoff erhalten. Das Rohprodukt wird in Essigsäure rekristallisiert, um grüne Kristalle zu erhalten. 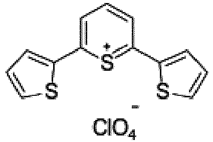
Summenformel: C₁₃H₉ClO₄S₃ (359,94 g/mol)
Ausbeute: 3,0 g (8,3 mmol, 28 %)
ESI-MS: m/z 261 [M-ClO₄]⁺
Absorption (DCM): αₘₐₓ = 514 nm (ε = 31902 Lmol⁻¹cm⁻¹)
¹H-NMR (500 MHz, Acetonitril, ppm): δ = 8.55 (t, J = 8.8 Hz, 1H), 8.43 (d, J = 8.7 Hz, 2H), 8.20-8.04 (m, 4H), 7.42 (dd, J = 4.9, 4.0 Hz, 2H).
¹³C-NMR (125,75 MHz, Acetonitril, ppm): δ = 162.24, 151.35, 139.07, 137.80, 134.81, 132.50, 130.26.

### Synthesevorschrift für 2,2',6,6'-Tetrathienyl-4,4'-dithiobispyranyliden (Referenz)

Unter Schutzgasatmosphäre werden 0,65 ml (2,6 mmol, 1 Äq.) Tributylphosphin zu einer violetten Suspension aus 0,95 g (2,6 mmol, 1 Äq.) 2,6-Dithienylthiopyryliumperchlorat in 50 ml getrocknetem Acetonitril gegeben. Die Mischung ändert die Farbe zu grau und wird für 2,5 h bei Raumtemperatur gerührt. Anschließend werden 2,2 ml (13,0 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird als schwarzer Feststoff nach Filtration und Rekristallisation aus DMSO erhalten. 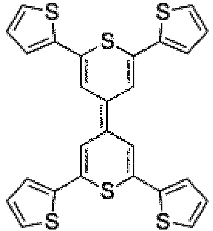
Summenformel: C₂₆H₁₆S₆ (519,96 g/mol)
Ausbeute: 0,26 g (0,5 mmol, 39 %)
ESI-MS: m/z 520 [M]⁺
Absorption (DMF): αₘₐₓ = 512 nm
Schmelzpunkt: 314 °C
¹H-NMR (500 MHz, Pyridin, ppm): δ = 7.52 (dd, J = 5.1, 1.1 Hz, 1H), 7.50 1 7.46 (m, 1H), 7.40 (s, 1H), 7.13 (dd, J = 5.1, 3.7 Hz, 1H).

### Synthesevorschrift für 1,5-Di-(2-(5-methyl)thienyl)pentan-1,5-dion

15 ml wasserfreies Dichlormethan (DCM) wird unter Schutzgasatmosphäre zu 16,0 g (120 mmol, 2 Äq.) Aluminiumchlorid in einem 100 ml-Rundkolben gegeben. Tropfenweise wird eine Lösung aus 11,6 ml (120 mmol, 2,4 Äq.) 2-Methylthiophen und 6,4 ml (50 mmol, 1 Äq.) Glutarylchlorid in 15 ml DCM über 10 min zugegeben. Bei der Zugabe ändert sich die Farbe von hellorange zu dunkelrot. Die Lösung wird über Nacht gerührt und der Kolben in einem Eisbad abgekühlt. Die Reaktion wird mittels Eis und konzentrierter Salzsäure (2 ml) gestoppt. Unter Rühren wird Wasser zugegeben bis die exotherme Reaktion mit dem Überschuss Aluminiumchlorid beendet ist. Die Mischung wird mit 50 ml DCM verdünnt und für 2 h gerührt. Die organische Phase wird mit warmen DCM extrahiert, über Magnesiumsulfat getrocknet und unter Vakuum konzentriert. Das Rohprodukt wird zermahlen und mit kaltem Diethylether gewaschen. 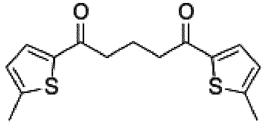
Summenformel: C₁₅H₁₆O₂S₂ (292,06 g/mol)
Ausbeute: 9,9 g (33,8 mmol, 68 %)
ESI-MS: m/z 293 [M]⁺
¹H-NMR (500 MHz, CDCl₃, ppm): δ = 7.53 (d, J = 3.7 Hz, 1H), 6.76 (m, 1H), 2.94 (t, J = 7.0 Hz, 2H), 2.51 (d, J = 0.7 Hz, 3H), 2.13 (p, J = 7.0 Hz, 1H).
¹³C-NMR (125,75 MHz, CDCl₃, ppm): δ = 192.47, 149.60, 141.96, 132.64, 126.74, 37.75, 19.72, 15.60.

### Synthesevorschrift für 2,6-Di-(2-(5-methyl)thienyl)pyryliumtetrafluoroborat

9,7 ml (76,2 mmol, 10 Äq.) Tetrafluorborsäurelösung (50 % (m/m) in Wasser) werden tropfenweise über 30 Minuten zu einer Suspension aus 2,2 g (7,6 mmol, 1 Äq.) 1,5-Di-(2-(5-methyl)thienyl)pentan-1,5-dion in 50 ml Essigsäureanhydrid gegeben, während die Temperatur mittels eines Eisbads unterhalb 15°C gehalten wird. Nachdem die Zugabe abgeschlossen ist, wird die Mischung für weitere 2 h bei Raumtemperatur gerührt und über Nacht bei 5°C stehen gelassen. Nach der Zugabe von 500 ml Hexan/Diethylether (1:10) fällt ein roter Niederschlag aus. Das Produkt wird durch Vakuumfiltration, Waschen mit Diethylether und Vakuumtrocknung bei Raumtemperatur erhalten. 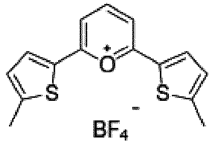
Summenformel: C₁₅H₁₃BF₄OS₂ (360,04 g/mol)
Ausbeute: 1,0 g (2,8 mmol, 37 %)
ESI-MS: m/z 273 [M-BF₄]⁺, 305 [M-BF₄+CH₃OH]⁺
Absorption (DCM): αₘₐₓ = 520 nm (ε = 27691 Lmol⁻¹cm⁻¹)
¹H-NMR (500 MHz, Acetonitril, ppm): δ = 8.45 (t, J = 8.4 Hz, 1H), 8.06 (d, J = 4.0 Hz, 2H), 7.88 (d, J = 8.4 Hz, 2H), 7.13 (dd, J = 4.0, 0.9 Hz, 2H), 2.66 (s, 6H).
¹³C-NMR (75 MHz, Acetonitril, ppm): δ = 166.03, 157.53, 154.45, 137.45, 131.13, 130.83, 116.23, 16.58.

### Synthesevorschrift für 2,2',6,6'-Tetra-( 2-methylthienyl)-4,4'-bispyranyliden

Unter Schutzgasatmosphäre werden 1,2 ml (4,7 mmol, 1 Äq.) Tributylphosphin zu einer orangen Suspension aus 1,69 g (4,7 mmol, 1 Äq.) 2,6-Di-(2-(5-methyl)thienyl)pyryliumtetrafluoroborat in 50 ml getrocknetem Acetonitril gegeben. Die Mischung ändert die Farbe zu gelb und wird für 2,5 h bei Raumtemperatur gerührt. Anschließend werden 4,0 ml (23,5 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird als schwarzer Feststoff nach Filtrieren, Waschen mit Acetonitril und Trocknen an Luft erhalten. 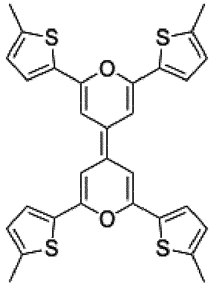
Summenformel: C₃₀H₂₄O₂S₄ (544,07 g/mol)
Ausbeute: 0,89 g (1,64 mmol, 70 %)
HR-El-MS: m/z 544.0661 [M]⁺
Absorption (DMF): αₘₐₓ = 488 nm (ε = 44663 Lmol⁻¹cm⁻¹)
Schmelzpunkt: 328 °C
¹H-NMR (600 MHz, Benzol, ppm): δ = 7.16 (s, 1H), 6.45 (d, J = 3.0 Hz, 1H), 6.40 (br.s., 1H), 2.11 (br.s., 3H).

### Synthesevorschrift für 2,6-Di(5-methylthienyl)thiopyryliumperchlorat

In einen 250 ml-Rundkolben werden sukzessiv 5,00 g (17,1 mmol, 1,0 Äq.) 1,5-Di-(2-(5-methyl)thienyl)pentan-1,5-dion, 5,72 g (25,7 mmol, 1,5 Äq.) Phosphor(V)-sulfid, 250 ml Essigsäure und 10,90 g (102,2 mmol, 6,0 Äq.) Lithiumperchlorat eingebracht. Die Mischung wird für 3 h unter Rückfluss gekocht. Die Farbe wechselt von orange zu tiefviolett. Die Mischung wird heiß filtriert und für 48 h stehen gelassen. Die grünen Kristalle werden mit Diethylether gewaschen und an Luft getrocknet 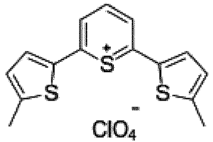
Summenformel: C₁₅H₁₃ClO₄S₃ (387,97 g/mol)
Ausbeute: 2,16 g (5,6 mmol, 33 %)
ESI-MS: m/z 289 [M-ClO₄]⁺
Absorption (DCM): αₘₐₓ = 552 nm (ε = 37946 Lmol⁻¹cm⁻¹)
¹H-NMR (500 MHz, Acetonitril, ppm): δ = 3.33 (dd, J = 9.5, 3.1 Hz, 1H), 3.25 4 3.15 (m, 2H), 7.93 (d, J = 4.0 Hz, 2H), 7.13 (dd, J = 4.0, 1.0 Hz, 2H), 2.65 (s, 6H).
¹³C-NMR (75 MHz, Acetonitril, ppm): δ = 161.09, 156.02, 150.44, 135.56, 135.27, 131.57, 123.49, 16.76.

### Synthesevorschrift für 2,2',6,6'-Tetra(2-methylthienyl)-4,4'-dithiobispyranyliden

Unter Schutzgasatmosphäre werden 0,7 ml (2,8 mmol, 1 Äq.) Tributylphosphin zu einer violetten Suspension aus 1,10 g (2,8 mmol, 1 Äq.) 2,6-Di(5-methylthienyl)thiopyryliumperchlorat in 50 ml getrocknetem Acetonitril gegeben. Die Mischung ändert die Farbe zu grau und wird für 2,5 h bei Raumtemperatur gerührt. Anschließend werden 2,4 ml (14,0 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird als schwarzer Feststoff nach Filtration und Rekristallisation aus DMSO erhalten. 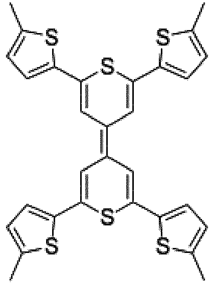
Summenformel: C₃₀H₂₄S₆ (576,02 g/mol)
Ausbeute: 0,58 g (1,0 mmol, 72 %)
ESI-MS: m/z 576 [M]⁺
Absorption (DMF): αₘₐₓ = 521 nm
Schmelzpunkt: 304 °C
¹H-NMR (500 MHz, Pyridin, ppm): δ = 7.35 (m, 1H), 7.29 (d, J = 3.6 Hz, 1H), 6.76 (dd, J = 3.6, 1.1 Hz, 1H), 2.33 (s, 3H).

### Synthesevorschrift für 1,5-Di-(2-(5-ethyl)thienyl)pentan-1,5-dion

15 ml wasserfreies Dichlormethan (DCM) wird unter Schutzgasatmosphäre zu 16,0 g (120 mmol, 2 Äq.) Aluminiumchlorid in einem 100 ml-Rundkolben gegeben. Tropfenweise wird eine Lösung aus 12,7 ml (120 mmol, 2,4 Äq.) 2-Ethylthiophen und 6,4 ml (50 mmol, 1 Äq.) Glutarylchlorid in 15 ml DCM über 10 min zugegeben. Bei der Zugabe ändert sich die Farbe von hellorange zu dunkelrot. Die Lösung wird über Nacht gerührt und der Kolben in einem Eisbad abgekühlt. Die Reaktion wird mittels Eis und konzentrierter Salzsäure (2 ml) gestoppt. Unter Rühren wird Wasser zugegeben bis die exotherme Reaktion mit dem Überschuss Aluminiumchlorid beendet ist. Die Mischung wird mit 50 ml DCM verdünnt und für 2 h gerührt. Die organische Phase wird mit warmen DCM extrahiert, über Magnesiumsulfat getrocknet und unter Vakuum konzentriert. Das Rohprodukt wird zermahlen und aus Diethylether rekristallisiert. 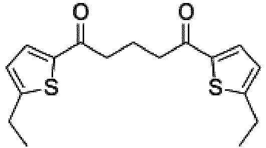
Summenformel: C₁₇H₂₀O₂S₂ (320,09 g/mol)
Ausbeute: 10,3 g (32,1 mmol, 64 %)
ESI-MS: m/z 321 [M]⁺
¹H-NMR (500 MHz, CDCl₃, ppm): δ = 7.55 (d, J = 3.8 Hz, 1H), 6.79 (dt, J = 3.8, 0.9 Hz, 1H), 2.95 (t, J = 7.0 Hz, 2H), 2.85 (qd, J = 7.5, 0.6 Hz, 2H), 2.14 (p, J = 7.0 Hz, 1H), 1.30 (t, J = 7.5 Hz, 3H). ¹³C-NMR (75 MHz, CDCl₃, ppm): δ = 192.52, 157.16, 141.46, 132.47, 124.90, 37.76, 24.00, 19.74, 15.53.

### Synthesevorschrift für 2,6-Di-(2-(5-ethyl)thienyl)pyryliumtetrafluoroborat

9,7 ml (76,2 mmol, 10 Äq.) Tetrafluorborsäurelösung (50 % (m/m) in Wasser) werden tropfenweise über 30 Minuten zu einer Suspension aus 2,4 g (7,6 mmol, 1 Äq.) 1,5-Di-(2-(5-ethyl)thienyl)pentan-1,5-dion in 50 ml Essigsäureanhydrid gegeben, während die Temperatur mittels eines Eisbads unterhalb 15°C gehalten wird. Nachdem die Zugabe abgeschlossen ist, wird die Mischung für weitere 2 h bei Raumtemperatur gerührt und über Nacht bei 5°C stehen gelassen. Nach der Zugabe von 500 ml Hexan/Diethylether (1:10) fällt ein roter Niederschlag aus. Das Produkt wird durch Vakuumfiltration, Waschen mit Diethylether und Vakuumtrocknung bei Raumtemperatur erhalten. 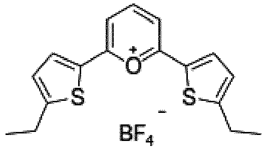
Summenformel: C₁₇H₁₇BF₄OS₂ (388,07 g/mol)
Ausbeute: 1,4 g (3,6 mmol, 48 %)
ESI-MS: m/z 301 [M-BF₄]⁺
Absorption (DCM): αₘₐₓ = 524 nm (ε = 31596 Lmol⁻¹cm⁻¹)
¹H-NMR (500 MHz, Acetonitril, ppm): δ = 8.45 (t, J = 8.4 Hz, 1H), 8.11 (d, J = 4.1 Hz, 2H), 7.89 (d, J = 8.4 Hz, 2H), 7.20 (dt, J = 4.1, 0.9 Hz, 2H), 3.05 (q, J = 7.5 Hz, 4H), 1.39 (t, J = 7.5 Hz, 6H). ¹³C-NMR (75 MHz, Acetonitril, ppm): δ = 165.95, 164.53, 154.10, 137.05, 130.22, 129.13, 115.99, 24.79, 15.56.

### Synthesevorschrift für 2,2',6,6'-Tetra-( 2-ethylthienyl)-4,4'-bispyranyliden

Unter Schutzgasatmosphäre werden 1,3 ml (5,2 mmol, 1 Äq.) Tributylphosphin zu einer orangen Suspension aus 2,0 g (5,2 mmol, 1 Äq.) 2,6-Di-(2-(5-ethyl)thienyl)pyryliumtetrafluoroborat in 60 ml getrocknetem Acetonitril gegeben. Die Mischung ändert die Farbe zu gelb und wird für 2,5 h bei Raumtemperatur gerührt. Anschließend werden 4,4 ml (26 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird als schwarzer Feststoff nach Filtrieren, Waschen mit Acetonitril und Trocknen an Luft erhalten. 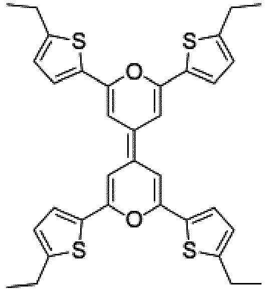
Summenformel: C₃₄H₃₂O₂S₄ (600,13 g/mol)
Ausbeute: 1,0 g (1,67 mmol, 64 %)
HR-El-MS: m/z 600.1288 [M]⁺
Absorption (DMF): αₘₐₓ = 488 nm (ε = 51749 Lmol⁻¹cm⁻¹)
Schmelzpunkt: 221 °C ¹H-NMR (500 MHz, Benzol, ppm): δ = 7.16 (s, 1H), 6.52 (d, J = 3.5 Hz, 1H), 6.44 (br.s., 1H), 2.53 (br.s., 2H), 1.07 (t, J = 7.6 HZ, 3H).

### Synthesevorschrift für 2,6-Di(5-ethylthienyl)thiopyryliumperchlorat

In einen 250 ml-Rundkolben werden sukzessiv 4,00 g (12,6 mmol, 1,0 Äq.) 1,5-Di-(2-(5-ethyl)thienyl)pentan-1,5-dion, 4,21 g (18,9 mmol, 1,5 Äq.) Phosphor(V)-sulfid, 250 ml Essigsäure und 8,07 g (75,6 mmol, 6,0 Äq.) Lithiumperchlorat eingebracht. Die Mischung wird für 3 h unter Rückfluss gekocht. Die Farbe wechselt von orange zu tiefviolett. Die Mischung wird heiß filtriert und für 48 h stehen gelassen. Die grünen Kristalle werden mit Diethylether gewaschen und an Luft getrocknet. 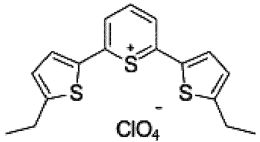
Summenformel: C₁₇H₁₇ClO₄S₃ (416,00 g/mol)
Ausbeute: 2,23 g (5,4 mmol, 43 %)
ESI-MS: m/z 317 [M-ClO₄]⁺
Absorption (DCM): αₘₐₓ = 554 nm (ε = 39270 Lmol⁻¹cm⁻¹)
¹H-NMR (300 MHz, Acetonitril, ppm): δ = 8.39 (dd, J = 9.6, 3.0 Hz, 1H), 8.26-8.16 (m, 2H), 7.95 (d, J = 4.0 Hz, 2H), 7.18 (dt, J = 4.0, 0.9 Hz, 2H), 3.01 (q, J = 7.5 Hz, 4H), 1.37 (t, J = 7.5 Hz, 6H). ¹³C-NMR (125,75 MHz, CDCl₃, ppm): δ = 161.92, 159.34, 149.72, 134.38, 133.70, 128.64, 126.94, 24.42, 15.28.

### Synthesevorschrift für 2,2',6,6'-Tetra(2-ethylthienyl)-4,4'-dithiobispyranyliden

Unter Schutzgasatmosphäre werden 0,6 ml (2,4 mmol, 1 Äq.) Tributylphosphin zu einer violetten Suspension aus 1,0 g (2,4 mmol, 1 Äq.) 2,6-Di(5-ethylthienyl)thiopyryliumperchlorat in 50 ml getrocknetem Acetonitril gegeben. Die Mischung ändert die Farbe zu grau und wird für 2,5 h bei Raumtemperatur gerührt. Anschließend werden 2,1 ml (12,0 mmol, 5 Äq.) N,N-Diisopropylethylamin zugegeben. Die Mischung wird unter Rückfluss bei 95°C für 2 h unter Schutzgasatmosphäre gekocht und über Nacht stehen gelassen. Das Produkt wird in Form von schwarzen Kristallen nach Filtration, Waschen mit Acetonitril und Trocknung an Luft erhalten. 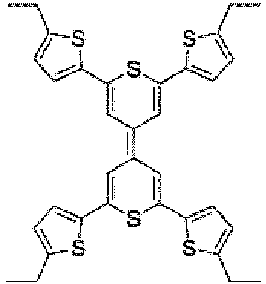
Summenformel: C₃₄H₃₂S₆ (632,08 g/mol)
Ausbeute: 0,46 g (0,73 mmol, 61 %)
ESI-MS: m/z 632 [M]⁺
Absorption (DMF): αₘₐₓ = 521 nm (ε = 72122 Lmol⁻¹cm⁻¹)
Schmelzpunkt: 253 °C
¹H-NMR (500 MHz, Pyridin, ppm): δ = 7.41 (s, 1H), 7.34 (d, J = 3.6 Hz, 1H), 6.80 (d, J = 3.6 Hz, 1H), 2.70 (q, J = 7.5 Hz, 2H), 1.17 (t, J = 7.5 Hz, 3H).
¹³C-NMR (125,75 MHz, Benzol, ppm): δ = 147.99, 139.99, 127.12, 125.02, 124.74, 124.42, 119.13, 24.16, 16.34.

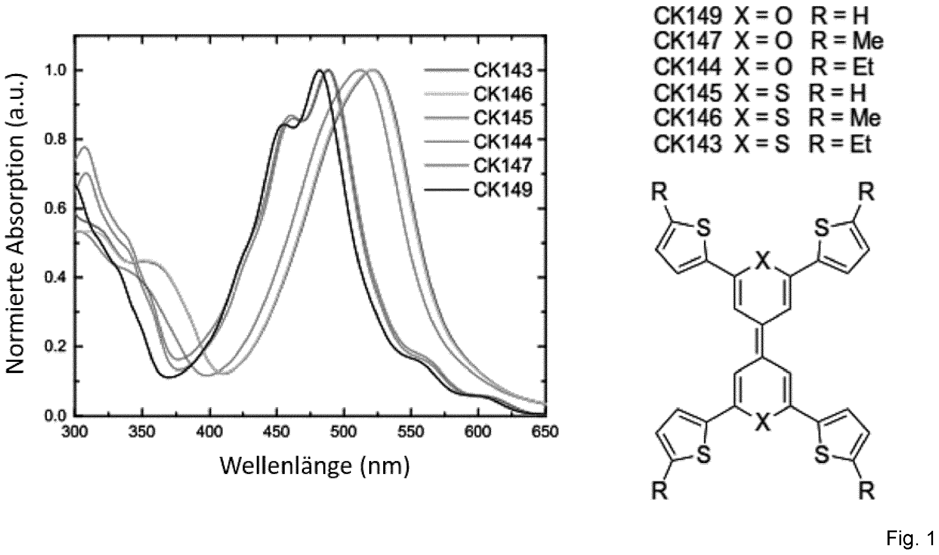

Fig. 1 zeigt die UV-Vis-Absorptionsspektren der Thienyl-substituierten Bispyranylidene und Dithiobispyranilidene in Dimethylformamid (DMF). Die Kurven sind jeweils auf den stärksten π-π*-Übergang normalisiert. Bei den Thienyl-substituierten Bispyranylidenen ist die Absorptionsbande geteilt in zwei Peaks und weist zwei rot-verschobene Schultern geringer Intensität auf. Die Thienyl-substituierten Dithiobispyranilidene weisen eine Absorptionsbande mit einem etwa 100 nm-rot-verschobenen λₘₐₓ im Vergleich zu den äquivalenten Thienyl-substituierten Bispyranylidenen auf. Weiterhin weisen die Methyl- bzw. Ethyl-substituierten Thienylverbindungen eine Rotverschiebung gegenüber den unsubstituierten Thienylverbindungen auf.

### Synthesevorschrift für 1,5-Bis(7-methyl-2,3-dihydrothieno[3,4-b][1,4]dioxin-5-yl])pentan-1,5-dion

2,33 g AlCl₃ werden mit 20 ml trockenem DCM unter intensivem Rühren suspendiert und mit Eis gekühlt. Unter Eiskühlung wird eine Lösung aus 2,72 g 5-Methyl-2,3-dihydrothieno[3,4-b][1,4]dioxin-5-yl, 1,4 g Glutarylchlorid und 20 ml DCM langsam zugetropft. Anschließend wird noch 24 h bei Raumtemperatur gerührt. 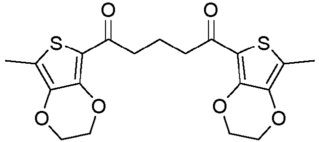
Summenformel: C₁₉H₂₀O₆S₂ (408,48 g/mol)
Ausbeute: 2,7g (80 %)
ESI-MS: m/z 409,1 [MH]⁺

### Synthesevorschrift für 2,6-Bis(7-methyl-2,3-dihydrothieno[3,4-b][1,4]dioxin-5-yl])-thiopyrylium-perchlorat

2,5 g 1,5-Bis(7-methyl-2,3-dihydrothieno[3,4-b][1,4]dioxin-5-yl])pentan-1,5-dion, 2,1g P₂S₁₀ und 3,9 g LiClO₄ werden in 80 ml Eisessig 3 h unter Rückfluß erhitzt. Nach dem Abkühlen und Stehen lassen über Nacht wird ein violetter Feststoff abgesaugt und mit Ether gewaschen. 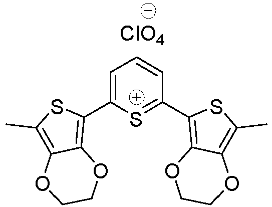
Summenformel: C₁₉H₁₇ClO₈S₃ (504,98 g/mol)
Ausbeute: 1,92 g (77 %)
ESI-MS: m/z 405,1 [M-ClO₄]⁺

### 2,2',6,6'-Tetrakis(7-methyl-2,3-dihydrothieno[3,4-b][1,4]dioxin-5-yl)-4,4'-dithiobispyraniliden

1,5 g des entsprechenden Thiopyryliumsalzes werden in 40 ml Acetonitril unter N₂-Spülung gelöst und 0,73 ml PBu₃ zugegeben. Diese Mischung wird 2 h bei Raumtemperatur gerührt, 1,5 ml Hünig-Base addiert und 2 h unter Rückfluss erhitzt. Nach dem Abkühlen bilden sich Kristalle, die abgesaugt und mit einem geeigneten Lösungsmittel gewaschen werden. 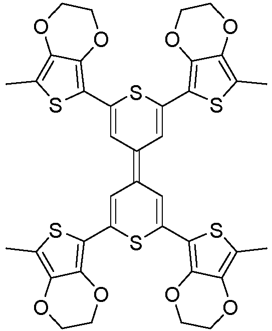
Summenformel: C₃₈H₃₂O₈S₆ (809,03 g/mol)
Ausbeute: 0,88 g (73 %)
ESI-MS: m/z 808,1 [M]⁺

### Allgemeine Vorschrift zur Synthese von Diselenobispyranilidene

Diselenobispyranilidene sind beispielsweise über Selenopyranthione aus Selenopyranone zugänglich (Detty *et al.* 1985). 10 mmol eines entsprechenden Selenopyranothions und 3 g Kupferpulver werden in 30 ml Toluen unter Rühren sowie Inertgas 16 h am Rückfluß erhitzt. Das Reaktionsgemisch wird einer geeigneten Aufarbeitung unterworfen und aus Acetonitril umkristallisiert.

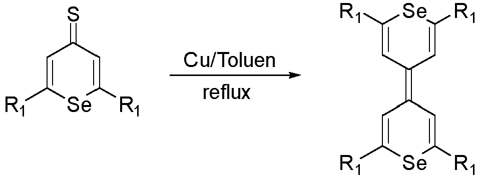

### Allgemeine Vorschrift zur Synthese von unsymmetrischen Bispyranilidenen

Unsymmetrische Bispyranilidene lassen sich analog den symmetrischen Verbindungen über Phosphoniumsalze in einer zweistufigen Reaktion nach Reynolds und Chen synthetisieren (Reynolds und Chen 1980). Es kann auch das Thio(Seleno)pyryliumsalz phosphoniliert werden, um danach mit dem Pyryliumsalz zur Reaktion gebracht zu werden.

Eine Lösung aus 10 mmol Pyryliumsalz in 25 ml Acetonitril wird 2 h bei Raumtemperatur gerührt bis die intensiv gelbe Farbe verschwunden ist. Der weiße Niederschlag wird abgesaugt und mit Acetonitril gewaschen.

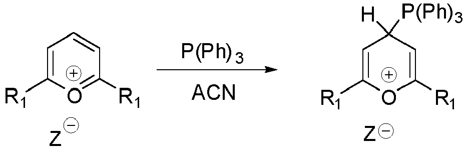

Im zweiten Schritt wird eine Suspension aus 2 mmol des entsprechenden zuvor dargestellten Phosphoniumsalzes in 35 ml THF unter Schutzgas unter Rühren auf-78°C gekühlt. 0,9 ml 2,5 M n-BuLi wird langsam zugegeben und weitere 5 min gerührt. Anschließend werden 2 mmol des gewünschten Thio- oder Selenopyryliumsalzes zugegeben und 1 h bei -78°C gerührt und danach 5 ml Triethylamin addiert. Die Reaktionsmischung wird über Nacht langsam auf Raumtemperatur erwärmt und chromatographisch gereinigt. Dabei können gute bis sehr gute Ausbeuten erreicht werden.

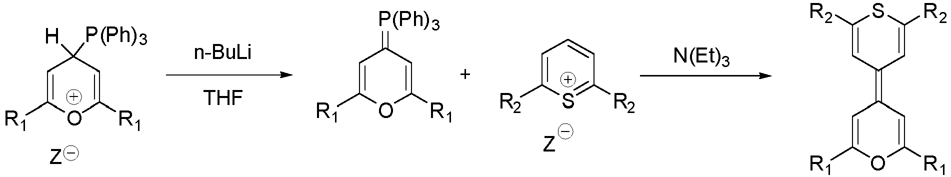

### Verfahren zur Trifluormethylierung

Negishi *et al.* beschreiben ein Verfahren zur Trifluormethylierung, womit die erfindungsgemäßen Verbindungen mit Perfluoroalkylresten hergestellt werden (Negishi *et al.* 2016).

### Elektrochemische Charakterisierung

### Zyklische Voltammetrie (Cyclic Voltammetry, CV)

Zur Messung der Oxidations- und Reduktionspotentiale und weitere Bestimmung der HOMO/LUMO-Energielevel wird zyklische Voltammetrie mittels eines Potentiostat (Methrom, µ-Autolab) und einer 3-Elektrodenzellkonfiguration durchgeführt. Als Elektrolyt wird Tetrabutylammoniumhexafluorophosphat in getrocknetem Dimethylformamid (DMF) oder Dimethylsulfoxid (DMSO) (0,1 M), als Arbeitselektrode eine Glas-Platin-Elektrode, als Gegenelektrode ein Platindraht und als Pseudo-Referenzelektrode wird Ag/AgCl verwendet. Als interner Standard wird Ferrocen/Ferrocenium zur Skalierung der gemessenen Potentiale verwendet. Sämtliche Lösungsmittel werden vor der Messung mit Stickstoff deoxygeniert. Die Messung erfolgt im Bereich von -1 V bis 1 V, mit einer Scanrate von 50 mV/s und 100 mV/s. Die Probe wird mit einer Konzentration von 1 mM/I gemessen.

### Dynamische Differenzkalorimetrie (differential scanning calorimetry, DSC)

Die thermische Charakterisierung erfolgt mittels dynamischer Differenzkalorimetrie (DSC) zur Bestimmung von Phasenübergängen, Schmelz- und Zersetzungstemperaturen. DSC wird mittels Mettler-Toledo DSC 1 Star und einer Scanrate von 5 K/min unter einer Stickstoffatmosphäre durchgeführt.

### Sublimation

Die Verbindungen werden zwei- bis dreimal zur Erhöhung der Reinheit kristallisiert. Die Sublimation erfolgt mittels 3-Zonen-Gradienten-Ofen der Firma VEB Hochvakuum Dresden.

### Herstellung optoelektronisches Bauelement

Das optoelektronische Bauelement wird zum Beispiel durch thermische Verdampfung unter Ultrahochvakuum (8 bis 10 mbar) auf ein Glassubstrat mit vorstrukturiertem ITO-Kontakt (Thin Film Devices, USA) hergestellt. Darauf wird eine Schicht 4, 7-Diphenyl-1,10-phenanthrolin (BPhen):Cs, C60, 2,2',6,6'-Tetrathienyl-4,4'-dithiobispyranyliden oder 2,2',6,6'-Tetra(2-methylthienyl)-4,4'-dithiobispyranyliden:C60 (Mischung 5 % (m/m) in C60), N4,N4'-Bis(9,9-dimethyl-9H-265-fluoren-2-yl)-N4,N4'-diphenylbiphenyl-4,4'-diamin (BF-DPB):F6-TCNNQ, 2,2'-(Perfluoronaphthalen-2,6-diylidene)dimalononitril (F6-TCNNQ) und Al. Das Bauteil ist gekennzeichnet durch die geometrische Überlappung des unteren und oberen Kontakts mit 6,44 m². Der organische Bereich wird mit einem kleinen Glassubstrat verklebt.

### Sensitive externe Quanteneffizienzen (EQE)-Messung

Die Messung der externen Quanteneffizienzen erfolgt mittels einer monochromatischen Lichtquelle, um einen Strom in einer organischen Solarzelle (OSC) unter Kurzschlussbedingungen zu erzeugen. Der resultierende Strom wird vorverstärkt und mittels eines Lock-in-Verstärkers (Signal Recovery 7280 DSP) analysiert.

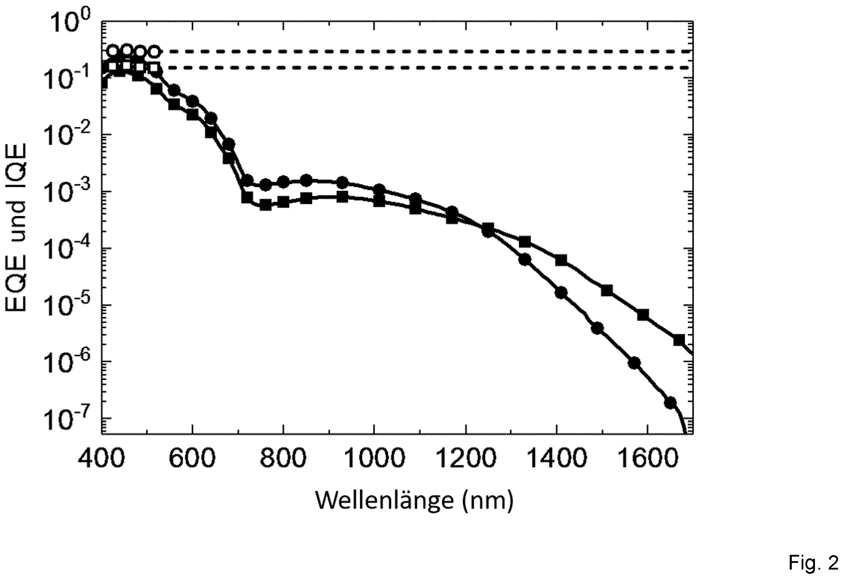

**Fig. 2** zeigt die Ergebnisse der Messung der externen Quanteneffizienz (EQE) und internen Quanteneffizienz (IQE) der optoelektronischen Bauteile umfassend Mischungen von 2,2',6,6'-Tetrathienyl-4,4'-dithiobispyranyliden (Referenz) (Kreise) bzw. 2,2',6,6'-Tetra(2-methylthienyl)-4,4'-dithiobispyranyliden (Quadrate) und C₆₀. Die IQE wurde im Spektralbereich von 425 nm bis 525 nm berechnet (IQE = EQE • Absorption ⁻¹) und eine mittlere IQE berechnet unter der Annahme, dass die IQE unabhängig von der Anregungswellenlänge ist.

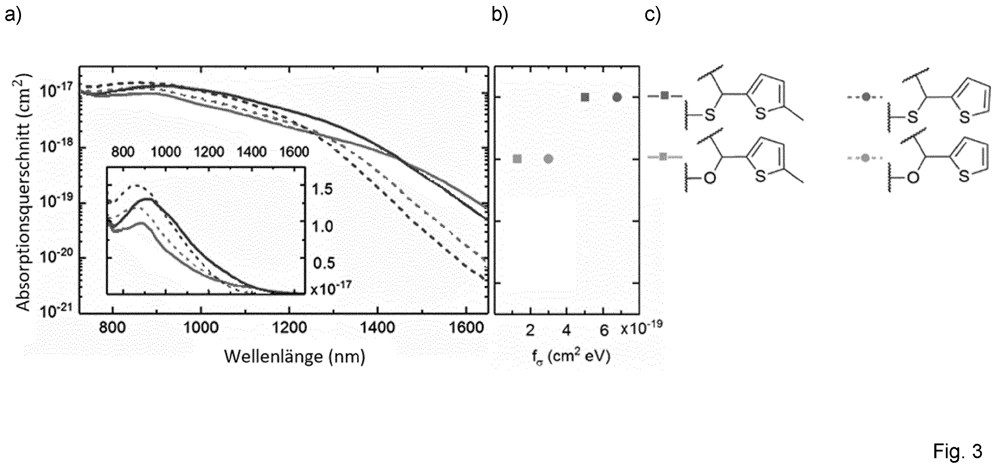

Unter der Verwendung der IQE und der Dichte der Donormoleküle in den 50 nm-dünnen Schichten, werden die EQE-Spektren in die Absorptionsprofile *σ*_{CT} **(****Fig. 3****)** umgerechnet. Die methylierten Verbindungen zeigen eine Rot-verschobene CT-Absorption im Vergleich zu den äquivalenten nicht-methylierten Verbindungen. Die Einführung von Schwefel in den Pyranilidenkern erhöht den Peak *σ*_{CT} und *f*_{σ} bis auf das Doppelte.

### Zitierte Nichtpatentliteratur

Bolag A, Mamada M, Nishida J, Yamashita Y (2009) Field-Effect Transistors Based on Tetraphenyldipyranylidenes and the Sulfur Analogues. Chem. Mater. 21, 4350-4352.
Detty MR, Hassett JW, Murray BJ, Reynolds GA (1985) Δ4,4-4-Chalcogenpyranyl-4H-Chalcogenapyrans. Tetrahedron 41, 4853-4859.
Fabre C, Fugnitto R, Strzelecka H (1976) Sur la synthèse de dipyranylidènes. Comptes rendus des séances de l'Académie des Sciences. Serie C, Sciences chimiques 282 (3), 175-177.
Mabon G, Cariou M, Simonet J (1989) The cathodic coupling of heterocyclic activat-ed thioketones. A new and efficient route to π-donors (I) - the synthesis of polysubstituted bipyranylidenes from 4H-pyran 4-thiones. New journal of chemistry 13 (8-9), 601-607.
Negishi K, Aikawa K, Mikami K (2016) Cyclic-Protected Hexafluoroacetone as an Air-Stable Liquid Reagent for Trifluoromethylations. European Journal of Organic Chemistry23, 4099-4104. Reynolds GA, Chen CH (1980) Synthesis of unsymmetrical Δ4,4-4-bi-4H-pyrans and thiopyrans. Journal of Organic Chemistry 45, 2458-2459.
Siegmund B, Mischok A, Benduhn J, Zeika O, Ullbrich S, Nehm F, Böhm M, Spoltore D, Fröb H, Körner C, Leo K, Vandewal K (2017) Organic narrowband near-infrared photodetectors based on intermolecular charge-transfer absorption. Nature Communications 8, 15421.
Strezelecka H, Schoenfelder W, Rivory J (1979) Electrical and optical properties of conducting TCNQ salts. Molecular Crystals and Liquid Crystals 52, 307-317.

## Patentansprüche

1. Verbindung gemäß Formel (I) 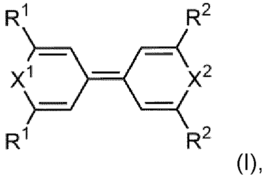 wobei
X¹ und X² jeweils unabhängig voneinander ausgewählt sind aus der Gruppe umfassend Sauerstoff, Schwefel und Selen,
R¹ und R² jeweils unabhängig voneinander ausgewählt sind aus der Gruppe umfassend 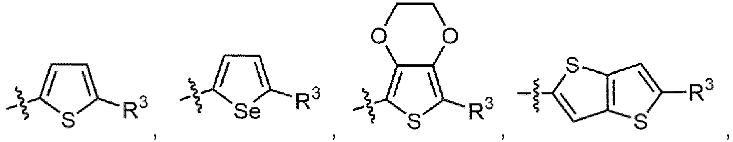 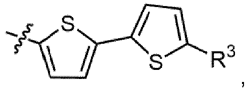
wobei R³ ausgewählt ist aus C1- bis C20-Alkylresten.

2. Verbindung nach Anspruch 1, **dadurch gekennzeichnet, dass** R³ ausgewählt ist aus der Gruppe umfassend Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl.

3. Verbindung nach Anspruch 1 oder 2, **dadurch gekennzeichnet, dass** R¹ und R² identisch sind.

4. Verbindung nach einem der Ansprüche 1 bis 3, **dadurch gekennzeichnet, dass** X¹ und X² jeweils unabhängig voneinander ausgewählt sind aus der Gruppe umfassend Schwefel und Selen.

5. Verbindung nach einem der Ansprüche 1 bis 4, **dadurch gekennzeichnet, dass** X¹ und X² identisch sind.

6. Verbindung nach einem der Ansprüche 1 bis 3, **dadurch gekennzeichnet, dass** X¹ und X² Sauerstoff und Schwefel, Sauerstoff und Selen oder Schwefel und Selen sind.

7. Verwendung mindestens einer Verbindung gemäß Formel (I) nach Anspruch 1, wobei X¹ und X² jeweils unabhängig voneinander ausgewählt sind aus der Gruppe umfassend Sauerstoff, Schwefel und Selen,
R¹ und R² jeweils unabhängig voneinander ausgewählt sind aus der Gruppe umfassend 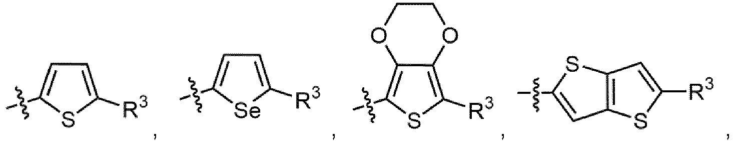 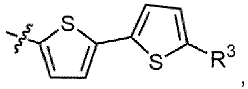
wobei R³ ausgewählt ist aus C1- bis C20-Alkylresten,
als Licht- oder IR-Absorber.

8. Verwendung nach Anspruch 7 in einem elektronischen oder optoelektronischen Bauelement.

9. Verwendung nach Anspruch 7 oder 8 als Donorabsorbermaterial in organischen Solarzellen *(organic solar cells,OSC),* als Lochtransportmaterial *(hole transport material,* HTM) in Grätzelzellen, in organischen integrierten Schaltungen *(organic integrated circuits,* O-IC), in organischen Feld-Effekt-Transistoren (OFETs), in organischen Dünnfilmtransistoren *(organic thin film transistor,* O-TFT), in organischen Leuchtdioden *(organic light emitting diode,* OLEDs), Photodetektoren oder in IR-Sensoren.

10. Elektronisches oder optoelektronisches Bauelement enthaltend mindestens eine Verbindung gemäß Formel (I) nach Anspruch 1, wobei
X¹ und X² jeweils unabhängig voneinander ausgewählt sind aus der Gruppe umfassend Sauerstoff, Schwefel und Selen,
R¹ und R² jeweils unabhängig voneinander ausgewählt sind aus der Gruppe umfassend 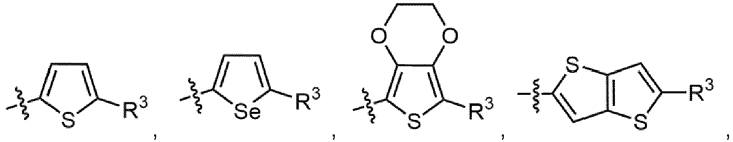 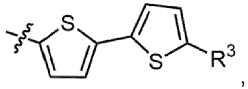
wobei R³ ausgewählt ist aus C1- bis C20-Alkylresten.

11. Elektronisches oder optoelektronisches Bauelement nach Anspruch 10 enthaltend mindestens eine weitere Verbindung,

12. Elektronisches oder optoelektronisches Bauelement nach Anspruch 11, **dadurch gekennzeichnet, dass** die mindestens eine weitere Verbindung eine Elektronenakzeptorverbindung ist.

## Claims

1. Compound according to formula (I) 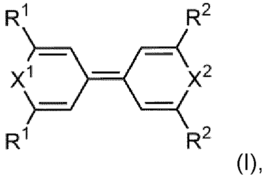 wherein
X¹ and X² are each independently selected from the group comprising oxygen, sulfur and selenium,
R¹ und R² are each independently selected from the group comprising 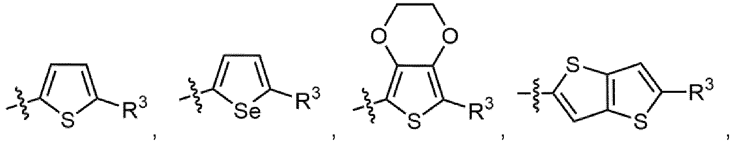 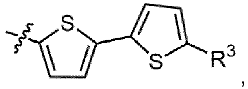
wherein R³ is selected from C1 to C20 alkyl residues.

2. Compound according to claim 1, **characterized in that** R³ is selected from the group comprising methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl.

3. Compound according to claim 1 or 2, **characterized in that** R¹ und R² are identical.

4. Compound according to any one of claims 1 to 3, **characterized in that** X¹ und X² are each independently selected from the group comprising sulfur and selenium.

5. Compound according to any one of claims 1 to 4, **characterized in that** X¹ und X² are identical.

6. Compound according to any one of claims 1 to 3, **characterized in that** X¹ und X² are oxygen and sulfur, oxygen and selenium, or sulfur and selenium.

7. Use of at least one compound according to formula (I) according to claim 1, wherein
X¹ and X² are each independently selected from the group comprising oxygen, sulfur and selenium,
R¹ und R² are each independently selected from the group comprising 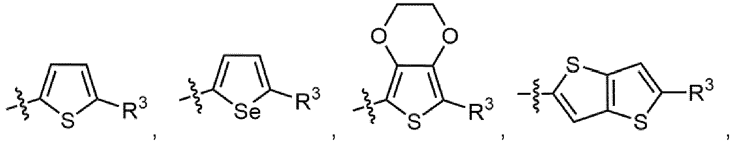 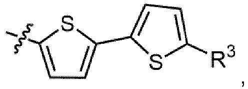
wherein R³ is selected from C1 to C20 alkyl residues,
as a light or IR absorber.

8. Use according to claim 7 in an electronic or optoelectronic component.

9. Use according to claim 7 or 8 as a donor absorber material in organic solar cells (OSC), as a hole transport material (HTM) in Grätzel cells, in organic integrated circuits (O-IC), in organic field-effect transistors (OFETs), in organic thin-film transistors (O-TFT), in organic light emitting diodes (OLEDs), in photodetectors or in IR sensors.

10. Electronic or optoelectronic component containing at least one compound according to formula (I) according to claim 1, wherein
X¹ and X² are each independently selected from the group comprising oxygen, sulfur and selenium,
R¹ und R² are each independently selected from the group comprising 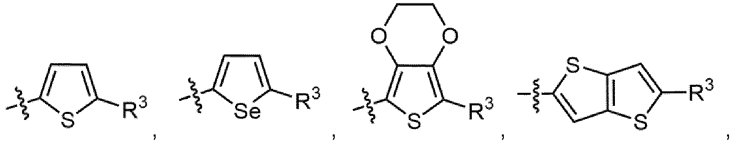 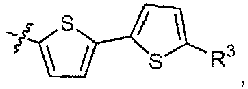
wherein R³ is selected from C1 to C20 alkyl residues.

11. Electronic or optoelectronic component according to claim 10 containing at least one further compound.

12. Electronic or optoelectronic component according to claim 11, **characterized in that** the at least one further compound is an electron-acceptor compound.

## Revendications

1. Composé selon la formule (I) 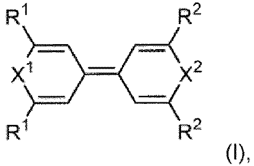 où
X¹ et X² sont respectivement indépendamment l'un de l'autre choisis dans le groupe comprenant oxygène, soufre et sélénium,
R¹ et R² sont respectivement indépendamment l'un de l'autre choisis dans le groupe comprenant 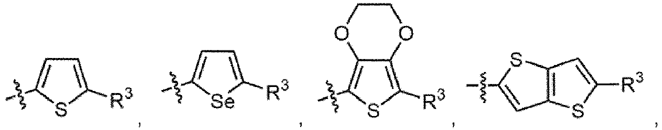 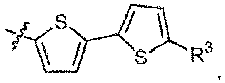
où R³ est choisi parmi les radicaux alkyle en C1 à C20.

2. Composé selon la revendication 1, **caractérisé en ce que** R³ est choisi dans le groupe comprenant méthyle, éthyle, propyle, butyle, pentyle, hexyle, heptyle, octyle, nonyle, décyle, undécyle, dodécyle, tridécyle, tétradécyle, pentadécyle, hexadécyle, heptadécyle, octadécyle, nonadécyle et eicosyle.

3. Composé selon la revendication 1 ou 2, **caractérisé en ce que** R¹ et R² sont identiques.

4. Composé selon l'une des revendications 1 à 3, **caractérisé en ce que** X¹ et X² sont respectivement indépendamment l'un de l'autre choisis dans le groupe comprenant soufre et sélénium.

5. Composé selon l'une des revendications 1 à 4, **caractérisé en ce que** X¹ et X² sont identiques.

6. Composé selon l'une des revendications 1 à 3, **caractérisé en ce que** X¹ et X² sont l'oxygène et le soufre, l'oxygène et le sélénium ou le soufre et le sélénium.

7. Utilisation d'au moins un composé conformément à la formule (I) selon la revendication 1, dans laquelle X¹ et X² sont respectivement indépendamment l'un de l'autre choisis dans le groupe comprenant oxygène, soufre et sélénium,
R¹ et R² sont respectivement indépendamment l'un de l'autre choisis dans le groupe comprenant 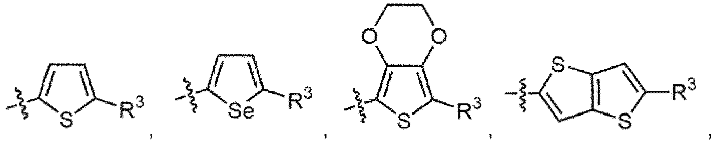 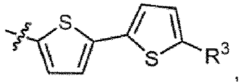
où R³ est choisi parmi les radicaux alkyle en C1 à C20,
en tant qu'absorbeur de lumière ou d'IR.

8. Utilisation selon la revendication 7 dans un composant électronique ou optoélectronique.

9. Utilisation selon la revendication 7 ou 8 en tant que matériau absorbant donneur dans des cellules solaires organiques *(organic solar cells,* OSC), en tant que matériau de transport de trous *(hole transport material,* HTM) dans des cellules de type Grätzel, dans des circuits intégrés organiques *(organic integrated circuits,* O-IC), dans des transistors organiques à effet de champ (OFET), dans des transistors organiques en couches minces *(organic thin film transistor, O-*TFT), dans des diodes électroluminescentes organiques *(organic light emitting diode,* DELO), des photodétecteurs ou dans des capteurs IR.

10. Composant électronique ou optoélectronique contenant au moins un composé conformément à la formule (I) selon la revendication 1, où
X¹ et X² sont respectivement indépendamment l'un de l'autre choisis dans le groupe comprenant oxygène, soufre et sélénium,
R¹ et R² sont respectivement indépendamment l'un de l'autre choisis dans le groupe comprenant 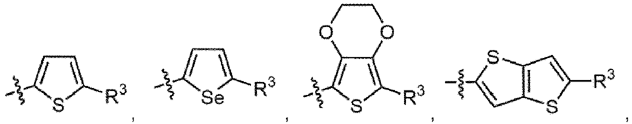 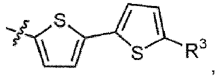
où R³ est choisi parmi les radicaux alkyle en C1 à C20.

11. Composant électronique ou optoélectronique selon la revendication 10, contenant au moins un autre composé,

12. Composant électronique ou optoélectronique selon la revendication 11, **caractérisé en ce que** l'au moins un autre composé est un composé accepteur d'électrons.
